# EUROPÄISCHE PATENTSCHRIFT

(11) **EP 1 572 037 B1**
(45) Veröffentlichungstag und Bekanntmachung des Hinweises auf die Patenterteilung: **07.05.2008**
(21) Anmeldenummer: 02782616.3
(22) Anmeldetag: 17.12.2002
(51) Int. Cl.: A61F 2/44

(54) **ZWISCHENWIRBELIMPLANTAT MIT KIPPBAREN GELENKTEILEN**
INTERVERTEBRAL IMPLANT WITH TILTABLE JOINT PARTS
IMPLANT INTERVERTEBRAL COMPRENANT DES PIECES D'ARTICULATION BASCULANTES

(43) Veröffentlichungstag der Anmeldung: 14.09.2005
(73) Patentinhaber: Synthes GmbH, 4436 Oberdorf (CH)
(72) Erfinder: AEBI, Max, Montreal, Quebec H3A 1A1 (CA); BURKARD, Dominique, CH-5014 Gretzenbach (CH); FRIGG, Robert, CH-2544 Bettlach (CH); LECHMANN, Beat, CH-2544 Bettlach (CH); MATHYS, Robert, Jr., CH-2544 Bettlach (CH); PAVLOV, Paul, NL-6523 NA Nijmegen (NL)
(74) Vertreter: Lusuardi, Werther
(86) Internationale Anmeldenummer: PCT/CH2002/000705
(87) Internationale Veröffentlichungsnummer: WO 2004/054476

(56) Entgegenhaltungen:
- WO-A-99/53871
- WO-A-99/59492
- WO-A-02/089701
- US-A- 5 893 889
- US-A1- 2002 052 656

## Beschreibung

Die Erfindung bezieht sich auf ein Zwischenwirbelimplantat gemäss dem Oberbegriff des Patentanspruchs 1 und auf ein Verfahren zum Ersetzen einer defekten, natürlichen Bandscheibe durch ein Zwischenwirbelimplantat gemäss dem Patentanspruch 22.

Nach Entfernung einer beschädigten, natürlichen Bandscheibe oder eines beschädigten Nukleus pulposus einer Bandscheibe werden Implantate oder Prothesen in den Zwischenwirbelraum zweier benachbarter Wirbelkörper eingebracht. Dabei entsteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Ein Zwischenwirbelimplantat gemäß dem Oberbegriff des Anspruch 1 wird in der WO 99/59492 offenbart. Ferner ist eine gattungsgemässe Bandscheibenendoprothese aus der DE-A 35 29 761 BÜTTNER bekannt. Diese bekannte Bandscheibenendoprothese besteht im wesentlichen aus zwei symmetrischen Abschlussplatten mit gegeneinander gerichteten konkaven Gleitflächen und je einer aussenstehenden Oberfläche zur Anlage an die Grundplatte, respektive die Deckplatte der angrenzenden Wirbelkörper und einem zwischen den Abschlussplatten positionierten Distanzstück mit zu den konkaven Gleitflächen an den Abschlussplatten komplementär ausgestalteten konvexen Gleitflächen. Die Gleitflächen sind in einer Ausführungsform als Teilflächen einer Zylindermantelfläche ausgebildet, wobei die an den beiden Abschlussplatten angeordneten Gleitflächen komplementär zu je einer der angrenzenden Gleitflächen am Distanzstück ausgestaltet sind und je zwei komplementäre Gleitflächen die aufeinander verschiebbaren Artikulationsflächen eines um eine Drehachse rotierbaren Gelenkteiles bilden. Das Gelenk umfasst ein oberes und ein unteres Gelenkteil mit je einer Drehachse. Die beiden Drehachsen sind um 90° zueinander versetzt. Nachteilig an dieser bekannten Bandscheibenendoprothese ist, dass
a) den durch die natürliche Bandscheibe übertragbaren überlagerten Schwenkbewegungen insbesondere bei anterior-posterior und lateraler Flexion, welche bei der natürlichen Bandscheibe unabhängig voneinander sind, durch die Ausgestaltung einer Bandscheibenendoprothese mit nur einem Drehzentrum nicht Rechnung getragen wird;
b) durch Scherbewegungen, insbesondere bei Translation in anterior-posteriorer Richtung das Wirbelgelenk (Facettengelenk) belastet wird, wodurch für den Patienten Schmerzen verursacht werden können;
c) nachteilige Reibungskräfte bei zwei aufeinander gleitbaren, artikulierenden Flächen entstehen. Ferner sind an den Flächen Verschleiss, d.h. unter anderem auch Abrieb sowie Widerstand bei der Bewegung der Gelenkteile die Folge. Zudem besteht das Risiko des "Stick-Slip" Effektes;
d) ein mechanischer Bandscheibenersatz die weitere Degeneration der betroffenen Bewegungssegmente kaum aufhalten kann. Das Wiederherstellen der ursprünglichen Bewegungsverhältnisse reduziert den Schmerz wesentlich und der Patient gewinnt an Lebensqualität. Bei neuem Auftreten von Schmerz muss jedoch eine Revision der Versorgung in Angriff genommen werden. Dabei wird üblicherweise eine Bandscheibenprothese nach herkömmlicher Bauart komplett entfernt und das Bewegungssegment versteift. Diese Operation belastet den Patienten ausserordentlich; und
e) der Form der Kontaktflächen zu den benachbarten Wirbelkörpern in der Regel nicht Rechnung getragen wird. Bandscheibenersatzimplantate herkömmlicher Bauart haben plane (flache) Kontaktflächen, welche oft noch mit kielartigen Erhebungen ergänzt sind.

Hier will die Erfindung Abhilfe schaffen. Der Erfindung liegt die Aufgabe zugrunde, ein Zwischenwirbelimplantat zu schaffen, welches ein Gelenk umfasst, dessen Gelenkachsen Lagerungen mit einer minimalen Reibung aufweisen.

Die Erfindung löst die gestellte Aufgabe mit einem Zwischenwirbelimplantat, welches die Merkmale des Anspruchs 1 aufweist und mit einem Verfahren zum Ersetzen einer defekten, natürlichen Bandscheibe durch ein Zwischenwirbelimplantat, welches die Schritte des Anspruchs 22 umfasst.

Die durch die Erfindung erreichten Vorteile sind im wesentlichen darin zu sehen, dass dank des erfindungsgemässen Zwischenwirbelimplantates
- Die Schwenkbewegungen in anterior-posteriorer Richtung und nach lateral unabhängig sind;
- die Reibfläche der Bewegungen auf klingenähnlichen Mitteln auf ein Minimum reduziert ist; und
- durch die infolge des Linienkontaktes zwischen den Gelenkteilen anstelle von Gleitflächen geringere Reibungskräfte im Gelenk auftreten und daher Relativbewegungen der Wirbelkörper, insbesondere die laterale Beugung und Flexions- / Extensionsbewegungen der Wirbelsäule nicht behindert werden.

Wegen der unterschiedlichen Positionen der natürlichen Drehachsen in den entlang der Wirbelsäule verschiedenen Bandscheibenräumen kann die Anordnung der Drehachsen windschief oder sich schneidend sein.

Durch die Länge der Klingen ergibt sich eine Stabilisierung der Gelenke gegen eine Abdrehen der beiden Teile um die Zentralachse. Die Flankenwinkel der Erhebungen betragen vorzugsweise zwischen 1° und 30° während die Flankenwinkel der Vertiefungen vorzugsweise zwischen 6° und 70° betragen.

In einer bevorzugten Ausführungsform des erfindungsgemässen Zwischenwirbelimplantates umfasst dieses zwei Gelenke mit je einem ersten und einem zweiten Gelenkteil, wobei das zweite Gelenkteil eine Vertiefung zur um die Drehachse kippbaren Aufnahme einer Erhebung am ersten Gelenkteil aufweist. Dadurch ist der Vorteil erreichbar, dass keine Translationsbewegung der an das Implantat angrenzenden Wirbelkörper zugelassen werden, wodurch die Facettengelenke geschont werden.

In einer anderen Ausführungsform des erfindungsgemässen Zwischenwirbelimplantates sind von den ventralen Seitenflächen her Mittel an den beiden Teilen anbringbar, wodurch die beiden Teile ventral auf einer bestimmten Distanz relativ zueinander gehalten werden können. Dadurch ist der Vorteil erreichbar, dass die beiden Teile zur Einführung in den Zwischenwirbelraum in eine Position mit fest gehaltener Höhe bringbar sind und nach der Einführung in den Zwischenwirbelraum um das Gelenk bewegbar und an die Grund- respektive Deckplatte der angrenzenden Wirbelkörper zur Anlage bringbar sind.

In einer weiteren Ausführungsform des erfindungsgemässen Zwischenwirbelimplantates ermöglichen die Mittel eine temporäre Blockierung der Beweglichkeit der beiden Teile um das Gelenk. Dadurch ist der Vorteil erreichbar, dass mittels eines minimal invasiven Eingriffes das im Zwischenwirbelraum integrierte Gelenk blockierbar ist. Dies ist besonders vorteilhaft in Fällen bei denen post-operativ Schmerzen auftreten, d.h. wo die Degeneration des betroffenen Wirbelsäulensegmentes weitergeht und der Chirurg eine Fusion der betroffenen Wirbel in Betracht zieht. Vorzugsweise sind die Mittel an den beiden ventralen Seitenflächen der beiden Teile anbringbar. Durch dieses spätere, sekundäre Blockieren der Bewegbarkeit der beiden Teile um das Gelenk wird das Zwischenwirbelimplantat versteift und in ein Arthrodesenimplantat (Fusions-Käfig) übergeführt.

In wiederum einer weiteren Ausführungsform des erfindungsgemässen Zwischenwirbelimplantates umfassen die Mittel einen Einsatz, welche in je eine Vertiefung an den einander gegenüberliegenden Oberflächen des oberen und unteren Teiles einsetzbar ist. Vorzugsweise sind die Vertiefungen als Schwalbenschwanzführungen ausgestaltet, welche an den ventralen Seitenflächen offen sind, so dass die zu den Schwalbenschwanzführungen komplementär ausgestalteten Enden des Einsatzes von ventral in die Schwalbenschwanzführungen eingeschoben werden können. Dadurch ist der Vorteil erzielbar, dass durch das Einführen des Einsatzes die Bewegbarkeit der beiden Teile um das Gelenk blockierbar ist. Die Starrheit der Blockierung lässt sich erhöhen, wenn die Schwalbenschwanzführungen so ausgestaltet sind, dass sie sich gegen die Zentralachse des Zwischenwirbelimplantates verjüngen, so dass der Einsatz zusätzlich in den Schwalbenschwanzführungen verkeilbar ist.

In wiederum einer anderen Ausführungsform des erfindungsgemässen Zwischenwirbelimplantates sind die beiden Teile mit Bohrungen zur Aufnahme von Knochenfixationsmittel, insbesondere von Knochenschrauben versehen, wobei die Bohrungen Längsachsen aufweisen, welche schräg zur Zentralachse stehen. Vorzugsweise durchdringen je zwei Bohrungen eines der beiden Teile von der ventralen Seitenfläche zur Appositionsfläche. Dabei können die Längsachsen, falls nur eine axiale Fixierung des Zwischenwirbelimplantates vorgesehen ist, nur von lateral betrachtet schräg zur Zentralachse stehen, oder falls eine winkelstabile Fixierung des Zwischenwirbelimplantates vorgesehen ist, auch von ventral betrachtet von den inneren Oberflächen der beiden Teile gegen die Appositionsflächen divergieren.

In einer weiteren Ausführungsform des erfindungsgemässen Zwischenwirbelimplantates sind die Bohrungen zur Aufnahme der Knochenfixationsmittel mit Innengewinden versehen, wodurch sich eine zusätzliche, rigide Fixierung der Knochenfixationsmittel in den beiden Teilen erreichen lässt. Vorzugsweise sind die Bohrungen konisch ausgestaltet, so dass durch die konischen Gewindeverbindungen zwischen den Innengewinden und den Aussengewinden an den Köpfen der Knochenfixationsmittel eine verstärkte Fixierung der Knochenfixationsmittel an jedem der beiden Teile erreichbar ist.

Die Appositionsflächen sind vorzugsweise konvex ausgestaltet und mit einer dreidimensionalen Strukturierung, vorzugsweise in Form von pyramidenförmigen Erhebungen versehen. Durch diese Ausgestaltung der Appositionsflächen wird der Anatomie der Wirbelkörperendplatten Rechnung getragen.

Der Ersatz einer defekten, natürlichen Bandscheibe durch ein Zwischenwirbelimplantat umfasst die Schritte:
A) blockieren des oder der Gelenke eines Zwischenwirbelimplantates mittels dafür vorgesehener Mittel in einer bestimmten Position des oder der Gelenke;
B) einführen des Zwischenwirbelimplantates in den zu behandelnden Zwischenwirbelraum;
C) lösen und entfernen der zur Blockierung des oder der Gelenke in das Zwischenwirbelimplantat eingesetzten Mittel. Durch die Blockierung des Gelenkes ist der Vorteil erreichbar, dass die beweglichen Teile mit den aussenstehenden Appositionsflächen einfacher in den zu behandelnden Zwischenwirbelraum einführbar sind.

In einer weiteren Anwendung des Verfahren umfasst dieses das nachträgliche Blockieren des oder der Gelenke am implantierten Zwischenwirbelimplantat mittels der zur Blockierung des oder der Gelenke vorgesehenen Mittel. Dadurch ist der Vorteil erreichbar, dass bei einem Auftreten von post-operativen Schmerzen für den Patienten oder bei einer weiteren Degeneration des betroffenen Bewegungssegmentes das oder die Gelenke am Zwischenwirbelimplantat postoperativ durch Einsetzen der dazu vorgesehenen Mittel blockierbar sind. Diese nachträgliche Blockierung ist mit einem minimal-invasiven, vorzugsweise einem lapraskopischen Eingriff möglich. Das Zwischenwirbelimplantat übernimmt dann die Aufgabe eines Käfigs, so dass das betroffene Bewegungssegment der Wirbelsäule versteift werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Ausführungsform des erfindungsgemässen Zwischenwirbelimplantates;
Fig. 2 eine perspektivische Ansicht der in Fig. 1 dargestellten Ausführungsform des erfindungsgemässen Zwischenwirbelimplantates in zusammengesetztem Zustand;
Fig. 3 eine Ansicht von lateral auf eine weitere Ausführungsform des erfindungsgemässen Zwischenwirbelimplantates;
Fig. 4 eine perspektivische Ansicht von ventral der Ausführungsform nach Fig. 3;
Fig. 5 ein Schnitt längs der Linie A-A in Fig. 1 mit Sicht von lateral; und
Fig. 6 ein Schnitt längs der Linie B-B in Fig. 1 mit Sicht von dorsal.

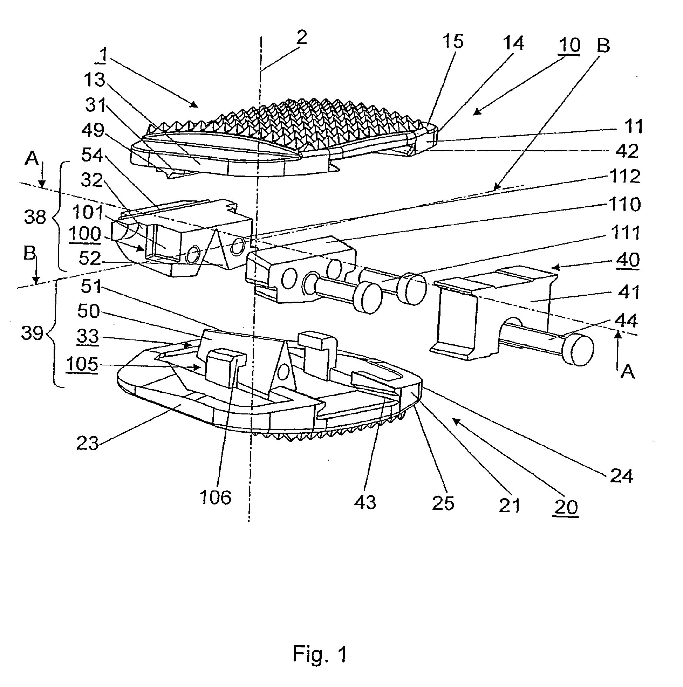

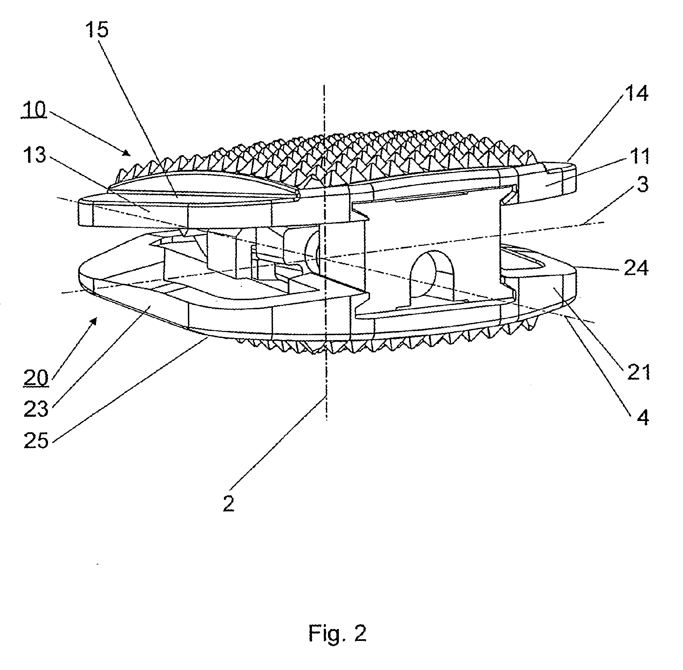

In den Fig. 1 und 2 ist eine Ausführungsform des erfindungsgemässen Zwischenwirbelimplantates 1 dargestellt, welche ein oberes Teil 10 mit einer oberen, quer zur Zentralachse 2 angeordneten Appositionsfläche 15 zur Anlage an die Grundplatte eines angrenzenden Wirbelkörpers, ein unteres Teil 20 mit einer unteren, quer zur Zentralachse 2 angeordneten Appositionsfläche 25 zur Anlage an die Deckplatte des angrenzenden Wirbelkörpers und zwei Gelenke 38;39 umfasst. Das obere Teil 10 und das untere Teil 20 sind über die Gelenke 38;39 relativ zueinander bewegbar verbunden, wobei die Bewegbarkeit des oberen Teils 10 relativ zum unteren Teil 20 um eine erste, quer zur Zentralachse 2 angeordnete Drehachse 3 innerhalb eines Winkelbereiches von +10° bis -6° eingeschränkt ist und um eine zweite, quer zur Zentralachse 2 und senkrecht zur ersten Drehachse 3 angeordneten Drehachse 4 innerhalb eines Winkelbereiches von ± 7° eingeschränkt ist.

Die Gelenke 38;39 sind durch drei Gelenkteile 31;32;33 realisiert, wovon das untere Gelenkteil 33 und das obere Gelenkteil 31 je ein mit dem mittleren Gelenkteil 32 zusammenwirkendes Gelenk 38;39 bilden. Die zwei Gelenke 38;39 sind als Wippen ausgestaltet und weisen je eine Drehachse 3;4 auf, wobei die Drehachsen senkrecht aufeinander und senkrecht zur Zentralachse 2 stehen. Das untere Gelenk 39 umfasst eine das untere Gelenkteil 33 bildende, mit dem unteren Teil 20 verbundene Erhebung 50 und eine im mittleren Gelenkteil 32 angeordnete, die Erhebung 50 aufnehmende Vertiefung 52. Die Erhebung ist mit einer die Drehachse 4 bildenden Kante 51 ausgestaltet, welche in der Vertiefung 52 derart gelagert ist, dass die beiden Gelenkteile 32;33 ein auf der Kante 51 um die Drehachse 4 kippbares Gelenk 39 bilden. Analog setzt sich das obere Gelenk 38 aus einer am oberen Gelenkteil 31 angeordneten zur Drehachse 3 parallelen Erhebung 49 und einer am mittleren Gelenkteil 32 angeordneten, die Erhebung 49 aufnehmenden Vertiefung 54 zusammen. Die Erhebung 49 ist mit einer die Drehachse 3 bildenden Kante 53 ausgestaltet, welche in der Vertiefung 54 derart gelagert ist, dass die beiden Gelenkteile 31;32 ein auf der Kante 53 um die Drehachse 3 kippbares Gelenk 38 bilden.

Die Bewegbarkeit der beiden Teile 10;20 relativ zueinander ist durch die Mittel 40 lösbar blockierbar. Die Mittel 40 umfassen in der hier dargestellten Ausführungsform einen von den ventralen Seitenflächen 11;21 der beiden Teile 10;20 her quer zur Zentralachse 2 und parallel zu den lateralen Seitenflächen 13;14;23;24 der beiden Teile 10;20 einschiebbaren Einsatz 41. Das Einschieben des Einsatzes 41 erfolgt in zwei Vertiefungen 42;43, welche als Schwalbenschwanzführungen ausgestaltet sind. Der Einsatz 41 wird von den ventralen Seitenflächen 11;21 der beiden Teile 10;20 in die als Schwalbenschwanzführungen ausgestalteten Vertiefungen 42;43 eingeführt und am unteren Teil 20 mittels einer Schraube 44 befestigt. Zudem ist der Einsatz 41 endständig komplementär zu den Vertiefungen 42;43 ausgestaltet, so dass die beiden Teile 10;20 bei eingeschobenem Einsatz 41 parallel zur Zentralachse 2 relativ zueinander fixiert sind.

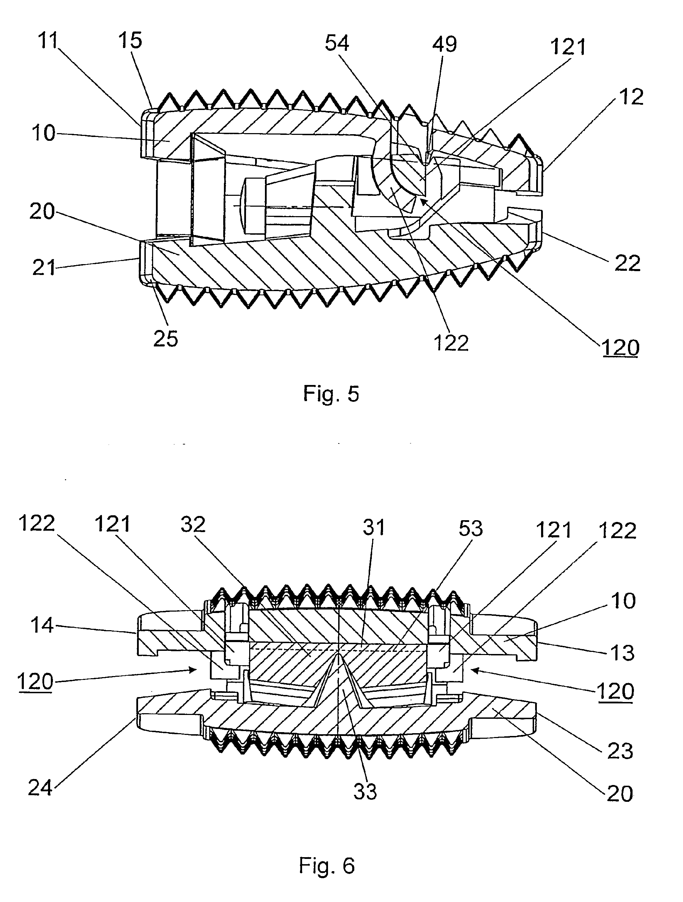

Ferner sind am mittleren Gelenkteil 32 erste Arretiermittel 100 angebracht, welche mit zweiten Arretiermitteln 105 am unteren Gelenkteil 33 in Eingriff bringbar sind und nach dem Befestigen des Fixierelementes 110 am mittleren Gelenkteil 32 ein Entfernen des mittleren Gelenkteiles 32 vom unteren Gelenkteil 33 verhindern. Die Befestigung des Fixierelementes 110 erfolgt mittels Schrauben 111, welche in die Gewindelöcher 112 neben der Vertiefung 52 in das mittlere Gelenkteil 32 eingeschraubt werden. Die ersten Arretiermittel 100 umfassen Vertiefungen 101 am mittleren Gelenkteil 32, welche mit den Nasen 106 der zweiten Arretiermittel 105 formschlüssig in Eingriff bringbar sind. Dadurch wird verhindert, dass die beiden Gelenkteile 32;33 voneinander getrennt werden können. Zwischen dem oberen Gelenkteil 31 und dem mittleren Gelenkteil 32 sind ferner Scharniere 120 (Fig. 5 und 6) angeordnet, wodurch die beiden Gelenkteile 31;32 parallel zur Zentralachse 2 zusammengehalten werden, ohne dass die Rotationsbewegung der beiden Gelenkteile 31;32 relativ zueinander um die erste Drehachse 3 dadurch eingeschränkt wird.

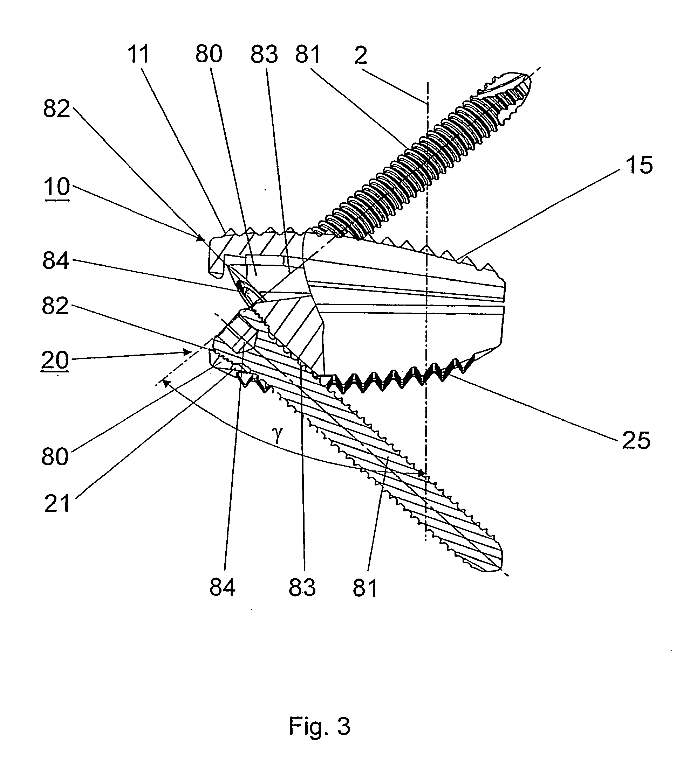

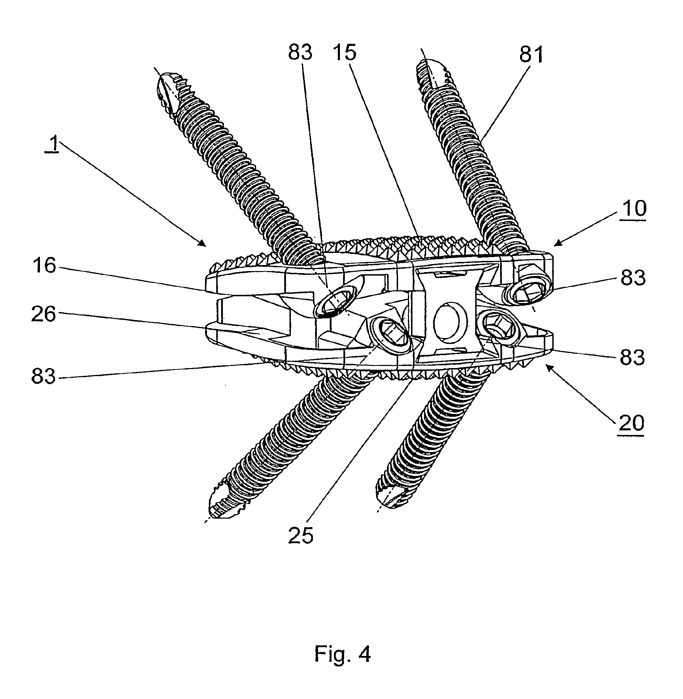

In Fig. 3 ist eine Ausführungsform des erfindungsgemässen Zwischenwirbelimplantates 1 dargestellt, welche sich von der in den Fig. 1 und 2 dargestellten Ausführungsform nur darin unterscheidet, dass die beiden Teilen 10;20 Bohrungen 80 zur Aufnahme von Knochenfixationsmitteln 81 umfassen, wobei die Knochenfixationsmittel 80 hier als Knochenschrauben ausgestaltet sind. Die Bohrungen 80 weisen Längsachsen 83 auf, welche einen Winkel γ mit der Zentralachse 2 einschliessen. Ferner durchdringen je zwei Bohrungen 80 (Fig. 4) eines der beiden Teile 10;20 von der ventralen Seitenfläche 11;21 zur Appositionsfläche 15;25. Die Längsachsen 83 der Bohrungen 80 stehen sowohl von lateral betrachtet (Fig. 3) als auch von ventral betrachtet (Fig. 4) schräg zur Zentralachse 2. Ferner sind die Bohrungen 80 konisch, sich gegen die Appositionsflächen 15;25 verjüngend ausgestaltet und mit Innengewinden 82 versehen, welche zur schraubbaren Aufnahme der mit komplementären Aussengewinden versehenen Schraubenköpfe 84 der als Knochenschrauben ausgestalteten Knochenfixationsmittel 81 dienen.

In den Fig. 5 und 6 sind die beiden Scharniere 120 zwischen dem oberen Gelenkteil 31 und dem mittleren Gelenkteil 32 ausführlicher dargestellt. Die Scharniere 120 gestatten eine relative Drehbewegung der beiden Gelenkteile 31;32 um die durch die Kante 53 an der zweiten Erhebung 49 und der Vertiefung 54 gebildete, erste Drehachse 3 (Fig. 2) und umfassen am mittleren Gelenkteil 32 lateral endständig angebrachte Nocken 121, welche gegen die ventralen Seitenflächen 11;21 des Zwischenwirbelimplantates 1 abgerundet sind, und am oberen Gelenkteil 31 angebrachte Schalen 122, welche die abgerundeten Seiten der Nocken 121 von den ventralen Seitenflächen 11;21 her kreisbogenförmig mit einem Winkel von ca. 90° umschliessen. Durch diese Schalen 122 wird das obere Gelenkteil 31 parallel zur Zentralachse 2 (Fig. 1) am mittleren Gelenkteil 32 festgehalten.

## Patentansprüche

1. Zwischenwirbelimplantat (1), insbesondere künstliche Bandscheibe, mit einer Zentralachse (2), einem oberen Teil (10), das für die Anlage an die Grundplatte eines darüber liegenden Wirbelkörpers geeignet ist und einem unteren Teil (20), das für die Anlage an die Deckplatte eines darunter liegenden Wirbelkörpers geeignet ist, wobei
A) das obere Teil (10) eine ventrale Seitenfläche (11), eine dorsale Seitenfläche (12), zwei laterale Seitenflächen (13,14), eine obere Appositionsfläche (15) und eine untere Oberfläche (16) aufweist;
B) das untere Teil (20) eine ventrale Seitenfläche (21), eine dorsale Seitenfläche (22), zwei laterale Seitenflächen (23,24), eine untere Appositionsfläche (25) und eine obere Oberfläche (26) aufweist;
C) die beiden Teile (10,20) durch zwei zwischen den beiden Teilen (10;20) angeordnete Gelenke (38;39) relativ zueinander bewegbar sind, wobei
D) jedes der Gelenke (38;39) eine Drehachse (3;4) aufweist und die beiden Drehachsen (3;4) quer zueinander angeordnet sind; und
E) die beiden Gelenke (38;39) durch ein mit dem oberen Teil (10) verbundenes, oberes Gelenkteil (31), ein mittleres Gelenkteil (32) und ein mit dem unteren Teil (20) verbundenes, unteres Gelenkteil (33) realisiert sind,
**dadurch gekennzeichnet, dass**
F) jedes Gelenk (38;39) ein erstes Gelenkteil (31;32;33) mit einer eine Kante (51;53) aufweisenden Erhebung (49;50) zur um die Drehachse (3;4) kippbaren Lagerung eines zweiten Gelenkteiles (31;32;33) umfasst.

2. Zwischenwirbelimplantat (1) nach Anspruch 1, **dadurch gekennzeichnet, dass** das untere Gelenkteil (33) eine die Kante (51) aufweisende Erhebung (50) zur um die Drehachse (4) kippbaren Lagerung des mittleren Gelenkteiles (31;32) umfasst.

3. Zwischenwirbelimplantat (1) nach Anspruch 1 oder 2, **dadurch gekennzeichnet, dass** das obere Gelenkteil (33) eine die Kante (53) aufweisende Erhebung (49) zur um die Drehachse (3) kippbaren Lagerung des mittleren Gelenkteiles (32) umfasst.

4. Zwischenwirbelimplantat (1) nach einem der Ansprüche 1 bis 3, **dadurch gekennzeichnet, dass** das zweite Gelenkteil (31;32;33) eine Vertiefung (52;54) zur Aufnahme der Erhebung (49;50) am ersten Gelenkteil (31;32;33) umfasst.

5. Zwischenwirbelimplantat (1) nach Anspruch 4, **dadurch gekennzeichnet, dass** das untere Gelenkteil (33) eine zur Drehachse (4) parallele Erhebung (50) mit einer die Drehachse (4) bildenden Kante (51) umfasst und die Erhebung (50) in der Vertiefung (52) am mittleren Gelenkteil (32) gelagert ist.

6. Zwischenwirbelimplantat (1) nach Anspruch 4 oder 5, **dadurch gekennzeichnet, dass** das obere Gelenkteil (31) eine zur Drehachse (3) parallele Erhebung (49) mit einer die Drehachse (3) bildenden Kante (53) umfasst und die Erhebung (49) in einer Vertiefung (54) am mittleren Gelenkteil (32) gelagert ist.

7. Zwischenwirbelimplantat (1) nach einem der Ansprüche 1 bis 6, **dadurch gekennzeichnet, dass** die Drehachsen (3;4) windschief zueinander stehen.

8. Zwischenwirbelimplantat (1) nach einem der Ansprüche 1 bis 7, **dadurch gekennzeichnet, dass** Mittel (40) vorgesehen sind, welche die beiden Teile (10;20), bei ihren ventralen Seitenflächen (11;21) gemessen, auf einer festen Distanz voneinander halten.

9. Zwischenwirbelimplantat (1) nach einem der Ansprüche 1 bis 7, **dadurch gekennzeichnet, dass** Mittel (40) vorgesehen sind, welche geeignet sind eine temporäre Blockierung der Beweglichkeit der beiden Teile (10,20) um die Gelenke (38;39) herbeizuführen.

10. Zwischenwirbelimplantat (1) nach Anspruch 8 oder 9, **dadurch gekennzeichnet, dass** die Mittel (40) an den beiden ventralen Seitenflächen (11,21) an den beiden Teilen (10;20) anbringbar sind.

11. Zwischenwirbelimplantat (1) nach Anspruch 9 oder 10, **dadurch gekennzeichnet, dass** die Mittel (40) einen Einsatz (41) mit einem unteren Ende (45) und einem oberen Ende (46) und an den beiden Teilen (10;20) je eine Vertiefung (42;43) in den Oberflächen (16;26) umfassen, welche an den ventralen Seitenflächen (11;21) offen sind, und dass der Einsatz (41) mit seinen Enden (45;46) in je eine Vertiefung (42;43) einfügbar ist.

12. Zwischenwirbelimplantat (1) nach Anspruch 11, **dadurch gekennzeichnet, dass** die Vertiefungen (42;43) Schwalbenschwanzführungen sind und die Enden (45;46) am Einsatz (41) komplementär zu diesen Schwalbenschwanzführungen ausgestaltet sind.

13. Zwischenwirbelimplantat (1) nach Anspruch 12, **dadurch gekennzeichnet, dass** sich die Schwalbenschwanzführungen von den ventralen Seitenflächen (11;21) her gegen die dorsalen Seitenflächen (12;22) verjüngen.

14. Zwischenwirbelimplantat (1) nach einem der Ansprüche 1 bis 13, **dadurch gekennzeichnet, dass** das obere und das untere Teil (10;20) je mindestens zwei von den ventralen Seitenflächen (11;21) zu den Appositionsflächen (15;25) durchgehende Bohrungen (80) mit Längsachsen (83) zur Aufnahme von Knochenfixationsmitteln (81) umfassen.

15. Zwischenwirbelimplantat (1) nach Anspruch 14, **dadurch gekennzeichnet, dass** die Längsachsen (83) der Bohrungen (80) mit der Zentralachse (2) einen Winkel γ einschliessen.

16. Zwischenwirbelimplantat (1) nach Anspruch 15, **dadurch gekennzeichnet, dass** der Winkel γ in einem Bereich von 20° und 65° liegt.

17. Zwischenwirbelimplantat (1) nach einem der Ansprüche 14 bis 16, **dadurch gekennzeichnet, dass** die Längsachsen (83) der Bohrungen (80) von den ventralen Seitenflächen (11;21) aus betrachtet von den inneren Oberflächen (16;26) gegen die Appositionsflächen (15;25) divergieren.

18. Zwischenwirbelimplantat (1) nach einem der Ansprüche 14 bis 17, **dadurch gekennzeichnet, dass** sich die Bohrungen (80) gegen die Appositionsflächen (15;25) konisch verjüngen.

19. Zwischenwirbelimplantat (1) nach einem der Ansprüche 14 bis 18, **dadurch gekennzeichnet, dass** die Bohrungen (80) ein Innengewinde (82) aufweisen.

20. Zwischenwirbelimplantat (1) nach einem der Ansprüche 1 bis 19, **dadurch gekennzeichnet, dass** das mittlere Gelenkteil (32) erste Arretiermittel (100) und das untere Gelenkteil (33) zweite Arretiermittel (105) umfasst, und dass die ersten und zweiten Arretiermittel (100;105) miteinander in Eingriff bringbar sind.

21. Zwischenwirbelimplantat (1) nach einem der Ansprüche 1 bis 20, **dadurch gekennzeichnet, dass** zwischen dem oberen Gelenkteil (31) und dem mittleren Gelenkteil (32) Scharniere (120) angebracht sind, wodurch die beiden Gelenkteile (31;32) parallel zur Zentralachse (2) zusammengehalten werden, ohne dass die Rotationsbewegung der beiden Gelenkteile (31;32) relativ zueinander um die erste Drehachse (3) **dadurch** eingeschränkt wird.

## Claims

1. Intervertebral implant (1), specifically an artificial intervertebral disk, with a central axis (2), an upper section (10), suitable for laying onto the base plate of a vertebral body lying above and a lower section (20) suitable for laying onto the cover plate of a vertebral body lying below, wherein
A) the upper section (10) is provided with a ventral side area (11), a dorsal side area (12), two lateral side areas (13,14), a top apposition surface (15) and a bottom surface (16);
B) the lower section (20) is provided with a ventral side area (21), a dorsal side area (22), two lateral side areas (23,24), a bottom apposition surface (25) and a top surface (26);
C) the two sections (10,20) are moveable in relation to each other by means of two joints (38;39) arranged between the two sections (10;20), wherein
D) each of the joints (38;39) is provided with a swivel axis (3;4) and the two swivel axes (3;4) are arranged transversely to each other; and
E) the two joints (38;39) are realised by means of an upper joint element (31) connected with the upper section (10), a central joint element (32) and a lower joint element (33) connected with the lower section (20),
**characterised in that**
F) each joint (38;39) comprising a first joint section (31;32;33) with an elevation (49;50) having an edge (51;53) for the bearing of a second joint section (31;32;33) in a way that allows tilting around the swivel axis (3;4).

2. Intervertebral implant (1) according to Claim 1, **characterised in that** the lower joint section (33) comprises an elevation (50) provided with an edge (51) for bearing of the central joint section (31;32) in a way that allows tilting around the swivel axis (4).

3. Intervertebral implant (1) according to Claim 1 or 2, **characterised in that** the upper joint element (33) comprises an elevation (49) provided with an edge (53) for bearing of the central joint section (32) in a way that allows tilting around the swivel axis (3).

4. Intervertebral implant (1) according to one of the claims 1 to 3, **characterised in that** the second joint section (31;32;33) comprises a depression (52;54) for receiving the elevation (49;50) on the first joint section (31 ;32;33).

5. Intervertebral implant (1) according to Claims 4, **characterised in that** the lower joint section (33) comprises an elevation (50) parallel to the swivel axis (4) with an edge (51) forming the swivel axis (4), and wherein this elevation (50) is carried in the depression (52) on the central joint section (32).

6. Intervertebral implant (1) according to Claim 4 or Claim 5, **characterised in that** the upper joint section (31) comprises an elevation (49) parallel to the swivel axis (3) with an edge (53) forming the swivel axis (3), and wherein the elevation (49) is carried in a depression (54) on the central joint section (32).

7. Intervertebral implant (1) according to one of the claims 1 to 6, **characterised in that** the swivel axes (3;4) are skewed in relation to each other.

8. Intervertebral implant (1) according to one of the claims 1 to 7, **characterised in that** a means (40) is provided that keeps the two sections (10;20), measured at their ventral side areas (11;21), at a fixed distance from each other.

9. Intervertebral implant (1) according to one of the claims 1 to 7, **characterised in that** a means (40) is provided that is suitable for causing temporary blocking of the mobility of the two sections (10,20) around the joints (38;39).

10. Intervertebral implant (1) according to Claim 8 or Claim 9, **characterised in that** the means (40) can be attached to the two ventral side areas (11,21) of the two sections (10;20).

11. Intervertebral implant (1) according to Claim 9 or Claim 10, **characterised in that** the means (40) comprises an insert (41) with a lower end (45) and an upper end (46) and a depression (42;43) in the surfaces (16;26) at each of the two sections (10;20), which are open on the ventral side areas (11;21), and that the insert (41) with its ends (45;46) can be inserted into each of the depressions (42;43).

12. Intervertebral implant (1) according to Claim 11, **characterised in that** the depressions (42;43) are dovetail guides and the ends (45;46) on the insert (41) are cnfigured complementary to these dovetail guides.

13. Intervertebral implant (1) according to Claim 12, **characterised in that** the dovetail guides are tapered from the ventral side areas (11;21) towards the dorsal side areas (12;22).

14. Intervertebral implant (1) according to one of the claims 1 to 13, **characterised in that** the upper and the lower sections (10;20) each comprises at least two bore holes (80) running through from the ventral side areas (11;21) to the apposition surfaces (15;25) with longitudinal axes (83) for receiving bone fixation devices (81).

15. Intervertebral implant (1) according to Claim 14, **characterised in that** the longitudinal axes (83) of the bore holes (80) enclose an angle γ with the central axis (2).

16. Intervertebral implant (1) according to Claim 15, **characterised in that** the angle γ is in a range of between 20° and 65°.

17. Intervertebral implant (1) according to one of the claims 14 to 16, **characterised in that** the longitudinal axes (83) of the bore holes (80) as seen from the ventral side areas (11;21) diverge from the inner surfaces (16;26) against the apposition surfaces (15;25).

18. Intervertebral implant (1) according to one of the claims 14 to 17, **characterised in that** the bore holes (80) are conically tapered towards the apposition surfaces (15;25).

19. Intervertebral implant (1) according to one of the claims 14 to 18, **characterised in that** the bore holes (80) are provided with an internal thread (82).

20. Intervertebral implant (1) according to one of the Claims 1 to 19, **characterised in that** the central joint section (32) comprises a first catching means (100) and the lower joint section (33) comprises a second catching means (105), and that the first and second catching means (100;105) can be engaged with each other.

21. Intervertebral implant (1) according to one of the Claims 1 to 20, **characterised in that** hinges (120) are attached between the upper joint section (31) and the central joint section (32), through which the two joint sections (31;32) are held together parallel to the central axis (2) without this causing any restriction of the rotational movement of the two joint sections (31;32) relative to each other around the first swivel axis (3).

## Revendications

1. Implant intervertébral (1), en particulier disque intervertébral artificiel, présentant un axe central (2), une partie supérieure (10), qui est appropriée à l'appui contre le plateau inférieur d'un corps vertébral sus-jacent, et une partie inférieure (20), qui est appropriée à l'appui contre le plateau supérieur d'un corps vertébral sous-jacent, dans lequel
A) la partie supérieure (10) présente une face latérale ventrale (11), une face latérale dorsale (12), deux faces latérales sur le côté (13, 14), une surface d'apposition supérieure (15) et une surface inférieure (16) ;
B) la partie inférieure (20) présente une face latérale ventrale (21), une face latérale dorsale (22), deux faces latérales sur le côté (23, 24), une surface d'apposition inférieure (25) et une surface supérieure (26) ;
C) les deux parties (10, 20) sont mobiles l'une par rapport à l'autre au moyen de deux articulations (38 ; 39) disposées entre les deux parties (10 ; 20), dans lequel
D) chacune des articulations (38 ; 39) présente un axe de rotation (3 ; 4) et les deux axes de rotation (3 ; 4) sont disposés transversalement l'un à l'autre ; et
E) les deux articulations (38 ; 39) sont formées par une partie d'articulation supérieure (31) reliée à la partie supérieure (10), une partie d'articulation intermédiaire (32) et une partie d'articulation inférieure (33) reliée à la partie inférieure (20),
**caractérisé en ce que**
F) chaque articulation (38 ; 39) comprend une première partie d'articulation (31 ; 32 ; 33) avec une protubérance (49 ; 50) présentant une arête (51 ; 53) pour monter de manière basculante autour de l'axe de rotation (3 ; 4) une deuxième partie d'articulation (31 ; 32 ; 33).

2. Implant intervertébral (1) selon la revendication 1, **caractérisé en ce que** la partie d'articulation inférieure (33) comprend une protubérance (50) présentant l'arête (51) pour monter de manière basculante autour de l'axe de rotation (4) la partie d'articulation intermédiaire (31 ; 32).

3. Implant intervertébral (1) selon la revendication 1 ou 2, **caractérisé en ce que** la partie d'articulation supérieure (33) comprend une protubérance (49) présentant l'arête (53) pour monter de manière basculante autour de l'axe de rotation (3) la partie d'articulation intermédiaire (32).

4. Implant intervertébral (1) selon l'une des revendications 1 à 3, **caractérisé en ce que** la deuxième partie d'articulation (31 ; 32 ; 33) comprend un évidement (52 ; 54) pour loger la protubérance (49 ; 50) au niveau de la première partie d'articulation (31 ; 32 ; 33).

5. Implant intervertébral (1) selon la revendication 4, **caractérisé en ce que** la partie d'articulation inférieure (33) comprend une protubérance (50) parallèle à l'axe de rotation (4) avec une arête (51) formant l'axe de rotation (4), et la protubérance (50) est logée dans l'évidement (52) au niveau de la partie d'articulation intermédiaire (32).

6. Implant intervertébral (1) selon la revendication 4 ou 5, **caractérisé en ce que** la partie d'articulation supérieure (31) comprend une protubérance (49) parallèle à l'axe de rotation (3) avec une arête (53) formant l'axe de rotation (3), et la protubérance (49) est logée dans un évidement (54) au niveau de la partie d'articulation intermédiaire (32).

7. Implant intervertébral (1) selon l'une des revendications 1 à 6, **caractérisé en ce que** les axes de rotation (3 ; 4) sont disposés de manière inclinée l'un par rapport à l'autre.

8. Implant intervertébral (1) selon l'une des revendications 1 à 7, **caractérisé en ce que** des moyens (40) sont prévus pour maintenir les deux parties (10 ; 20) à distance fixe l'une de l'autre, mesuré au niveau de leurs faces latérales ventrales (11 ; 21).

9. Implant intervertébral (1) selon l'une des revendications 1 à 7, **caractérisé en ce que** des moyens (40) sont prévus pour provoquer un blocage temporaire du mouvement des deux parties (10, 20) autour des articulations (38 ; 39).

10. Implant intervertébral (1) selon la revendication 8 ou 9, **caractérisé en ce que** les moyens (40) peuvent être placés sur les deux faces latérales ventrales (11, 21) des deux parties (10 ; 20).

11. Implant intervertébral (1) selon la revendication 9 ou 10, **caractérisé en ce que** les moyens (40) comprennent une pièce rapportée (41) présentant une extrémité inférieure (45) et une extrémité supérieure (46) et, au niveau des deux parties (10 ; 20), un évidement (42 ; 43) dans les surfaces (16 ; 26) qui sont ouvertes au niveau des faces latérales ventrales (11 ; 21), et **en ce que** la pièce rapportée (41) peut être insérée avec ses extrémités (45 ; 46) respectivement dans un évidement (42 ; 43).

12. Implant intervertébral (1) selon la revendication 11, **caractérisé en ce que** les évidements (42 ; 43) sont des guidages en queue d'aronde et les extrémités (45 ; 46) sont conçues, au niveau de la pièce rapportée (41), de manière complémentaire à ces guidages en queue d'aronde.

13. Implant intervertébral (1) selon la revendication 12, **caractérisé en ce que** les guidages en queue d'aronde se rétrécissent en partant des faces latérales ventrales (11 ; 21) jusqu'aux faces latérales dorsales (12 ; 22).

14. Implant intervertébral (1) selon l'une des revendications 1 à 13, **caractérisé en ce que** les parties supérieure et inférieure (10 ; 20) comprennent chacune au moins deux alésages (80) présentant des axes longitudinaux (83), s'étendant des faces latérales ventrales (11 ; 21) aux surfaces d'apposition (15 ; 25) pour recevoir des moyens de fixation osseuse (81).

15. Implant intervertébral (1) selon la revendication 14, **caractérisé en ce que** les axes longitudinaux (83) des alésages (80) forment un angle γ avec l'axe central (2).

16. Implant intervertébral (1) selon la revendication 15, **caractérisé en ce que** l'angle γ se situe dans une gamme de 20° à 65°.

17. Implant intervertébral (1) selon l'une des revendications 14 à 16, **caractérisé en ce que** les axes longitudinaux (83) des alésages (80), vu depuis les faces latérales ventrales (11 ; 21), divergent depuis les surfaces internes (16 ; 26) jusqu'aux surfaces d'apposition (15 ; 25).

18. Implant intervertébral (1) selon l'une des revendications 14 à 17, **caractérisé en ce que** les alésages (80) se rétrécissent de manière conique vers les surfaces d'apposition (15 ; 25).

19. Implant intervertébral (1) selon l'une des revendications 14 à 18, **caractérisé en ce que** les alésages (80) présentent un taraudage (82).

20. Implant intervertébral (1) selon l'une des revendications 1 à 19, **caractérisé en ce que** la partie d'articulation intermédiaire (32) comprend des premiers moyens d'arrêt (100) et la partie d'articulation inférieure (33) comprend des deuxièmes moyens d'arrêt (105), et **en ce que** les premiers et deuxièmes moyens d'arrêt (100 ; 105) peuvent être mis en prise les uns avec les autres.

21. Implant intervertébral (1) selon l'une des revendications 1 à 20, **caractérisé en ce que** des charnières (120) sont placées entre la partie d'articulation supérieure (31) et la partie d'articulation intermédiaire (32), si bien que les deux parties d'articulation (31 ; 32) peuvent être maintenues ensemble parallèlement à l'axe central (2) sans que cela limite le mouvement de rotation des deux parties d'articulation (31 ; 32) l'une par rapport à l'autre autour du premier axe de rotation (3).
